# EUROPEAN PATENT APPLICATION

(11) **EP 1 452 519 A1**
(43) Date of publication of application: **01.09.2004**
(21) Application number: 03472002.9
(22) Date of filing: 25.02.2003
(51) Int. Cl.: C07C 67/58, C07C 67/48, C07C 67/52, C07C 67/56, C07C 69/33

(54) **Method for the isolation and purification of pravastatin sodium**

(71) Applicant: Balkanpharma-Razgrad AD, 7200 Razgrad (BG)
(72) Inventor: Petkov, Nedelcho, 7200 Razgrad (BG); Nikolova, Antoniya, 7200 Razgrad (BG); Dimov, Dimcho, 7200 Razgrad (BG); Kostadinov, Milen, 7200 Razgrad (BG); Todorova, Dimitra, 7200 Razgrad (BG)
(74) Representative: Valcheva, Emiliya Nikolova

(57) **Abstract**

The invention relates to a method for the isolation and purification of pravastatin sodium, intended for application in the pharmaceutical industry. The application of the method results in pravastatin sodium salt of high yield and purity without recovery and additional purification of intermediate products. After the process the fermentationta broth is alkalized to pH 10.2, centrifuged, the resultant filtrate is extracted with butyl acetate and then reextracted with water. The obtained reextract is treated with sodium hydroxide to concentration of the hydroxide in the reextract of 0.5-4 %, and is again extracted with butyl acetate in the presence of sodium chloride. The precipitation of pravastatin is performed with t-octylamine. The obtained crystalline pravastatin-t-octylamine salt is converted into sodium salt, which is purified and recovered.

## Description

### Technical field

The invention relates to a method for the isolation and purification of pravastatin sodium, intended for the pharmaceutical industry.

### Prior art

Pravastatin is a HMG-CoA reduxidase inhibitor, a bioactive metabolite of mevastatin. It belongs to the group of statins and is used in human medicine as an antihyperchelesterolimic agent. The pharmaceutically acceptable pravastatin salt, used in medical practice is the sodium salt of the acid form with an open lactone ring. It is obtained by microbial hydroxylation of mevastatin(compactin). As a result of this process pravastatin is found in the fermentation broth in its acid form and partially as a lactone. The acid form of pravastatin and pravastatin lactone exhibit different polarities, which renders the process of recovery of purified pharmaceutical substance difficult. Moreover, the C-atom at position 6 is liable to epimerization and because of these significant quantities of 6-epi-pravastatin is formed during the microbial transformation.

A method for the recovery and purification of pravastatin is known, comprising acidification of the fermentation broth with trifluoracetic acid and extraction with ethyl acetate. The resultant extract is washed with a saturated solution of sodium chloride and is immediately treated with a 5 % aqueous solution of sodium hydrogen carbonate. The resultant aqueous solution, containing pravastatin sodium salt is acidified to pH 8 and is adsorbed on resin. It is eluted with a 50 % aqueous acetone, the eluate is concentrated and the sodium salt of pravastatin is crystallized by cooling (US 4448979).

A disadvantage of the method is the performing of crystallization in acetone in the presence of significant quantities of water, which reduces the yield and renders impossible the recovery of some impurities. The resultant product is with high water content and requires extensive drying.

A method for the production of pravastatin sodium salt is known, comprising filtration of the fermentation broth after microbial transformation, extraction of the filtrate with ethyl acetate at pH 3.5-4, the extract is washed with water, dried in anhydrous sodium sulphate and concentrated in vacuum. Pravastatin is transformed completely into lactone in the presence of catalytic quantities of trifluoracetic acid at ambient temperature and with continuous stirring. After the completion of the lactonization process, the ethyl acetate solution is washed with a solution of sodium hydrogen carbonate, and with water, the ethyl acetate is evaporated under vacuum, and the residue is purified by adsorption on a silicagel column and eluted from the column with an ethyl acetate-n-hexane mixture. The pravastatin lactone is hydrolyzed in acetone with sodium hydroxide to give a sodium salt, which is recrystallized in a mixture of ethanol-ethyl acetate (WO 0103647).

A method for the production of pravastatin is known, comprising filtration of mycelium after the microbial transformation and extraction of the resultant filtrate with ethyl acetate at pH 3.5-4. Dibenzylamine is added to the ethyl acetate extract, the ethyl acetate extract is concentrated at temperature 0-5ºC and the dibenzylammonium salt of pravastatin is precipitated. It is recovered and recrysrallized from acetone and is after that suspended in ethanol and 110 mol % of sodium hydroxide is added at ambient temperature and continuous stirring. Water is added to the alkaline solution, the pH of the solution is neutralized, and the ethanol is distilled under vacuum. The obtained aqueous concentrate is adsorbed on a polymer sorbent and eluted with an acetone-water mixture. The fractions, containing pravastatin are combined and after the removal of the acetone by evaporation under vacuum, and the aqueous residue are freeze-dried (WO0103647).

A disadvantage of the process is that the recovery step does not remove 6-epi-pravastatin, which requires complex fraction purification with adsorbing resins and silicagel.

A method for the production of pravastatin is known, comprising extraction of pravastatin from the fermentation broth after the fermentation with i-butyl acetate, re-extraction with water at pH 8.0-9.5 with ammonium hydroxide. The aqueous extract is acidified to pH 2.0-3.7 and pravastatin is extracted from it with i-butyl acetate. The resultant butyl acetate extract is dried with sodium sulphate and decolourized. When gaseous ammonia is passed through the solution, crystals of the ammonium salt of pravastatin are formed. The resultant ammonium salt is purified by at least one crystallization from a mixture of water-i-butyl acetate or water-acetone. The crystallization of the ammonium salt is induced by the addition of ammonium salt. The pravastatin ammonium salt crystals are removed by filtration, dried and dissolved in water. Pravastatin is extracted at pH 2-4 with i-butyl acetate in the acid form; it is then converted to sodium salt with intensive stirring and pulse addition of sodium hydroxide to pH 7.4-13. The solution of pravastatin sodium salt is treated with ion-exchange resin to remove the excess sodium hydroxide with intensive stirring until the pH of the solution reaches 7.4-7.8. After that the resin is filtered and the solution is concentrated under vacuum and is decolourized. Pravastatin sodium salt is crystallized by addition of acetonitrile and acetone and cooling at temperature - 10 ÷ 0ºC (WO 0230415).

A disadvantage of the method is the necessity for multiple steps in the production of pravastatin sodium salt, which reduces the yield and is a prerequisite for contamination with additional impurities. The direct extraction of the fermentation broth with an organic solvent contaminates the extract further. No method for selective removal of the impurities is applied.

### Description of the invention

The object of the invention is to provide a method for the preparation of pravastatin sodium salt with a high yield and purity using a simplified method scheme.

Using the method of this invention, the fermentation broth, obtained after the completion of the microbial transformation of mevastatin to pravastatin with a strain *Streptomyces roseochromogenes,* registered in the National Bank of Industrial Microorganisms and Cell Cultures of the Republic of Bulgaria under Nº 8072, is alkalized with stirring with sodium hydroxide to pH 10.2. The mycelium is centrifuged, suspended in water and centrifuged again. The combined filtrates, containing pravastatin, are acidified with nitric acid to pH 3-4, preferably 3.5-3.9 and are extracted twice with vigorous stirring with butyl acetate in a free acid form. The resultant butyl acetate extracts are combined and reextracted twice with water, at pH 8.5-11.0. Pravastatin is reextracted for 10-20 min. The temperature of the combined aqueous reextracts is raised to 30-32ºC and at this temperature 20% sodium hydroxide is added to a concentration of 0.5-4 % in the aqueous extract, preferably 1.5 %. The aqueous extract is maintained in these conditions for 85-90 min. The quantity of the 6'-epi-pravastatin impurity is monitored by HPLC testing until it reaches 0.2-3 %. Butyl acetate is added to the aqueous solution and the pH is adjusted to 4.1-4.3 with nitric acid. Then sodium chloride is added to the reaction mixture until its concentration reaches 10-20 %. Under these conditions pravastatin is extracted as an acid in butyl acetate. The butyl acetate extract is separated and the spent water is extracted again with butyl acetate. The extracts are combined, dried with disodium sulphate and decolourized with activated carbon. The solid phase is removed by filtration and t-octylamine is added to the butyl acetate filtrate, containing pravastatin acid, at a quantity 1.3-1.8 mol/mol pravastatin. The amine is added slowly over a period of 1-3 hours. After the first hour from the beginning of the amination step, the reaction mixture is seeded with pravastatin-t-octylamine salt. The crystallization continues for another 10-16 hours at temperature 0 ÷ -5ºC. The obtained crystal product is filtered, washed with acetone and dissolved in deionized water. The pH of the solution is adjusted to the range 10.5-13.0, preferably 11.0-11.5 with sodium hydroxide solution. After 10-20 min of stirring pravastatin-t-octylamine salt degrades and the resultant pravastatin sodium salt is purified and recovered.

For the purification and recovery of pravastatin sodium salt, butyl acetate is added to the aqueous solution, so that the released t-octylamine passes into the butyl acetate, while pravastatin sodium salt remains in the aqueous solution; the reaction mixture is left to separate. Strongly fused cathion-exchange resin, for example DIAION SK116 (sulphocathionite) is added to the separated aqueous layer until the pH is reduced to 8.1-8.5. After removing the resin by filtration, the obtained filtrate, containing pravastatin sodium salt is decolorized using activated carbon. The carbon is removed by filtration and the obtained filtrate is concentrated under vacuum. Pravastatin sodium salt is recovered and a lower aliphatic alcohol is added to the concentrate in a quantity of 2 mol/mol pravastatin. The solution is fed for 1 min to acetone, the quantity of acetone being determined in such a way that the concentration of the water after mixing is 2.0-3.0 %. The mixture is stirred for 3 hours at temperature 19-23ºC, and is left to rest for 16-20 hours at temperature 5 ÷ -5ºC. The obtained crystals of pravastatin sodium salt are filtered, washed with acetone and dried at 60ºC to reach water content in the product of less than 2.5 %.

Pravastatin sodium salt can be recovered in a stable crystalline form by dissolving the purified pravastatin sodium salt in water to obtain a 20-35 % solution, and 1 mol of lower alcohol is added to the solution. The resultant solution is lyophilized by freezing to temperature of -42 ÷ -45°C while the sublimation temperature is maintained at 5-15°C under vacuum of 100-250 mbar. The secondary drying is carried at 35°C to water content of 2 %.

The lower aliphatic alcohols, which can be used for the recovery of pravastatin sodium salt are: methanol, ethanol, propanol, i-propanol, butanol, i-butanol, amyl alcohol, i-amyl alcohol.

During the recovery of pravastatin sodium salt, the product crystallizes as a solvate of the lower aliphatic alcohol, and the resultant solvates degrade during the method crystallization in acetone or during lyophilization of the product.

The purification and recovery of pravastatin sodium salt is achieved by extracting the aqueous solution, obtained after the degradation of the pravastatin-t-octylamine salt with a solution of liquid ionite in butyl acetate. The liquid ionite, used for extraction is added in a quantity of 10-40 % of the amount of pravastatin in the solution. The extraction is performed at temperature of 10-30°C for 30-40 min, after that the butyl acetate layer is removed and the obtained aqueous layer is treated with aluminium oxide, decolorized with activated carbon and filtered. The filtrate is concentrated under vacuum and a lower aliphatic alcohol is added to it in a quantity of 2 mol/mol pravastatin. Pravastatin sodium salt is recovered by lyophilization with the temperature being reduced to -42 ÷ -45°C, and temperature of sublimation is maintained at at 5-15°C under vacuum 100-250 mbar. The secondary drying is performed at temperature of 35°C to water content less than 2 %.

The liquid ionites, used for the extraction of Pravastatin sodium salt are: Amberlit LA-1, Amberlit LA-2, Amberlit LA-3 and diisooctylphosphate.

The advantages of the method the invention are that a method for producing Pravastatin sodium salt was invented, granting high yield and purity and employing a simple method scheme without recovery and additional purification of the intermediate products.

### Explanation of the enclosed figures:

The Pravastatin content increases in the method of its purification after the method of this invention. The results from the HPLC testing at the individual steps are described in the enclosed figures as follows:

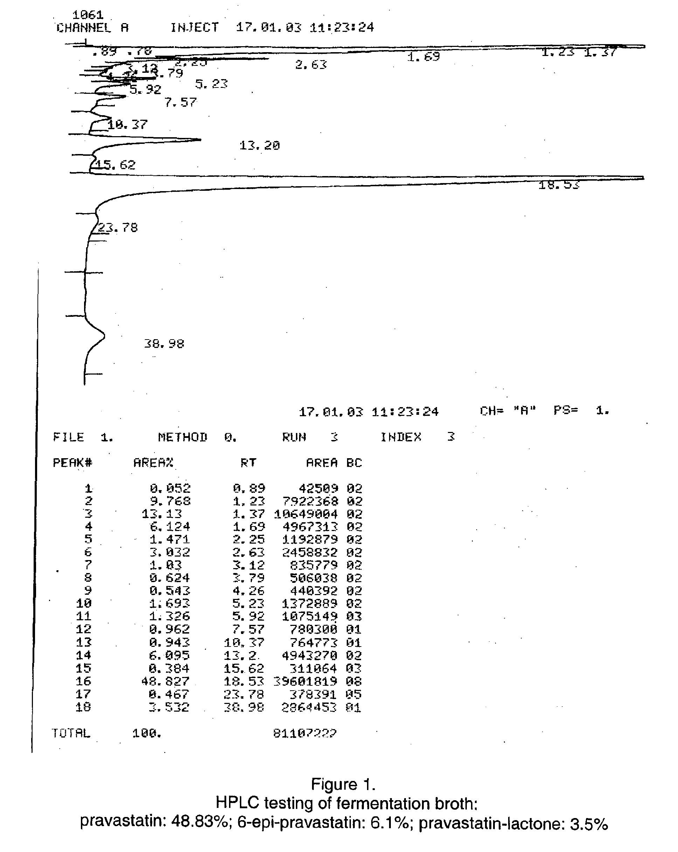

1. Figure 1. HPLC testing of fermentation broth, obtained during microbial transformation of Mevastatin to pravastatin with a strain of *Streptomyces roseochromogeues,* registered at the National Bank of Industrial Microorganisms and Cell Cultures of the Republic of Bulgaria under Nº 8072. Content of pravastatin: 48.83 %; 6-epi-pravastatin: 6.1 %; pravastatin lactone: 3.5 %.

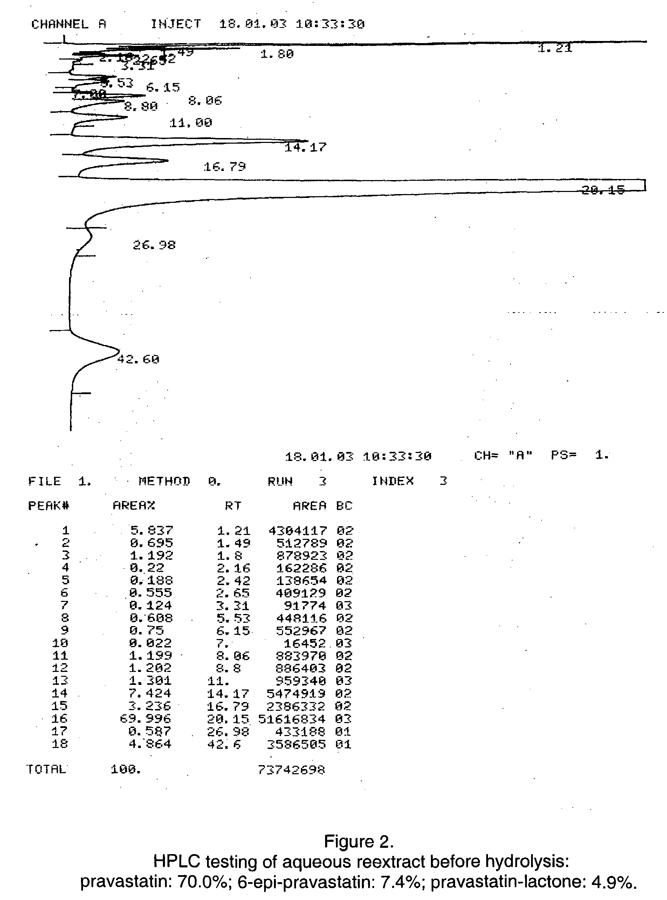

2. Figure 2. HPLC testing of aqueous reextract before hydrolysis: pravastatin: 70 %; 6-epipravastatin: 7.4 pravastatin lactone: 4.9 %.

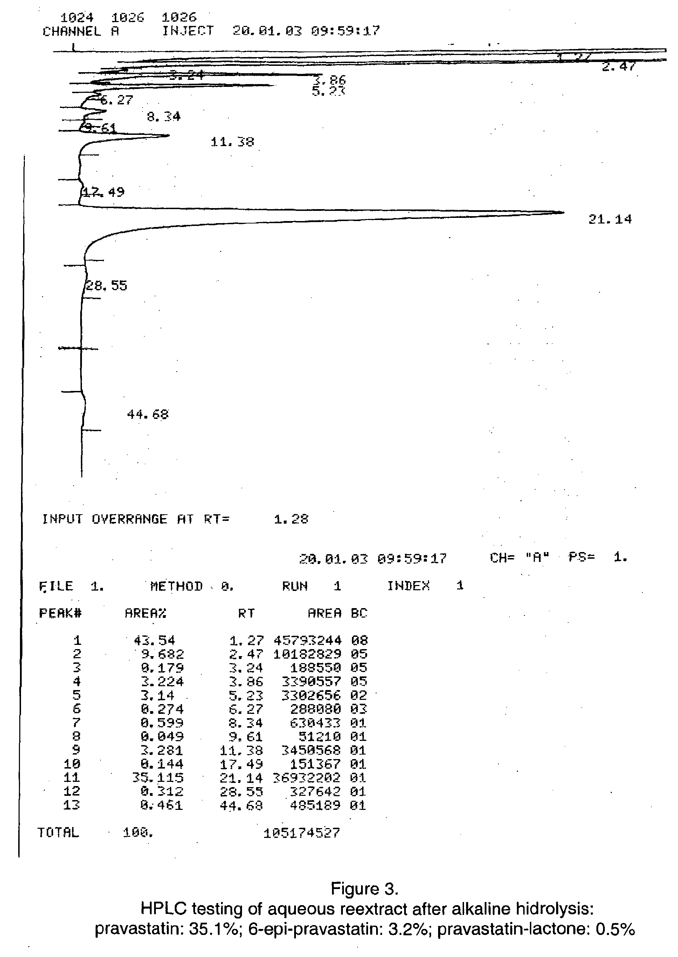

3. Figure 3: HPLC testing of aqueous reextract after alkaline hydrolysis: pravastatin: 35.1 %; 6-epi-pravastatin: 3.2 %; pravastatin lactone: 0,5 %.

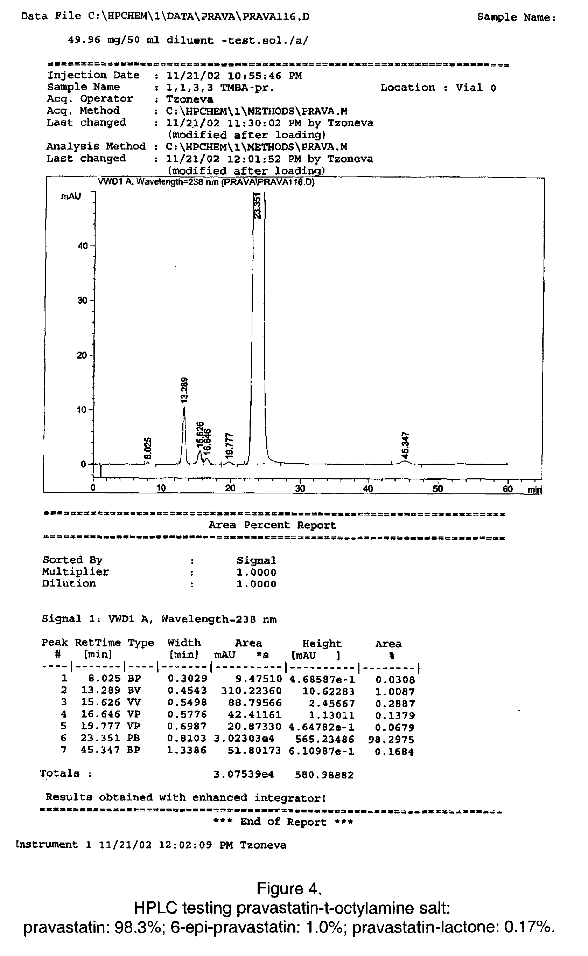

4. Figure 4: HPLC testing of pravastatin-t-octylamine salt: pravastatin: 98.3 %; 6-epi-pravastatin: 1.0 %; pravastatin lactone: 0.17 %.

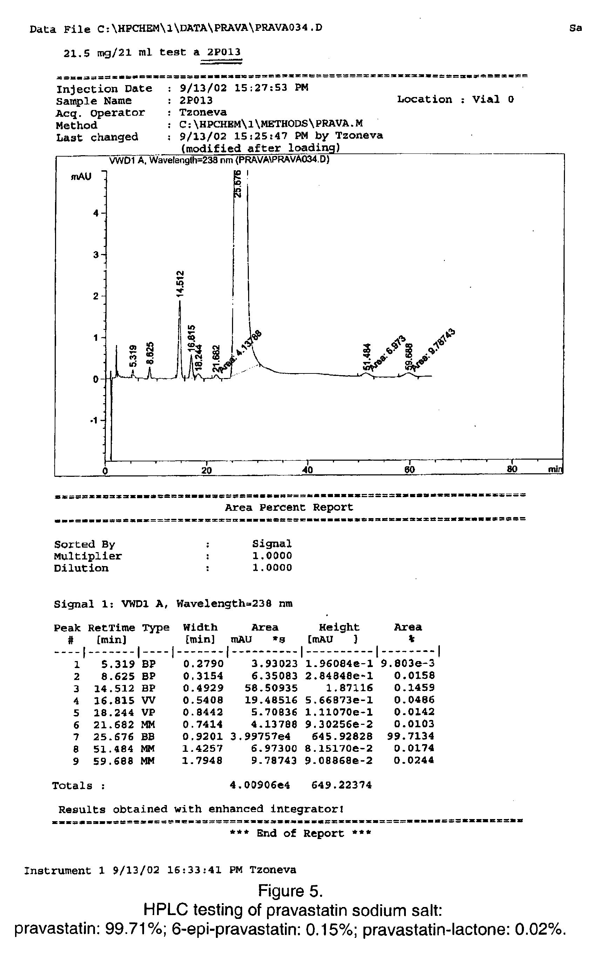

5. Figure 5: HPLC testing of pravastatin sodium salt, obtained using the method of the invention: pravastatin: 99.71 %; 6-epi-pravastatin: 0.15 %; pravastatin lactone: 0.02 %.

The enclosed HPLC test results for the individual steps of the method of the invention demonstrate that the method achieves transformation of the products at each processing step, the objective being their removal in the next step.

### Examples of the embodiment of the invention

The invention is illustrated by the following examples:

### Example 1

10% sodium hydroxide was added to the fermentation broth, containing 162.6 g pravastatin to pH 10.2. The broth is stirred for 30 min after the pH was stabilized and the mycelium was centrifuged. The mycelium was suspended in 40 l of water and centrifuged - 75 l of solution were obtained, containing 93 % of the initial activity. Butyl acetate (40 l) were added and the pH was adjusted to 3.7 with a 10 % nitric acid. The solution was stirred for 10 min and left to separate. Butyl acetate (40 l) were added to the aqueous layer and the extraction was repeated. Deionized water (4 l) were added to the combined extracts and the pH was adjusted to 9.6 with a 10 % sodium hydroxide. Pravastatin was reextracted for 10 min and the aqueous layer was separated, while the organic layer was reextracted with 0.5 l of water with the pH adjusted to 9.5 with sodium hydroxide. The two aqueous reextracts were combined (139.15 gA, yield 92 %). Sodium hydroxide (52 g as 20 % solution) were added to the reextracts and the temperature was increased to 31ºC and maintained for 85 min. The quantity of 6'-epi-pravastatin was reduced to 0.3 %. Butyl acetate (1.2 l) were added and using 10 % nitric acid the pH was adjusted to 4.1. Sodium chloride (730 g) were added and the solution was stirred for 10 min. The butyl acetate, containing pravastatin of 85 % purity was collected. The spent waters were extracted with 300 ml of butyl acetate. The two extracts were combined and dried with 75 g of sodium sulphate. The solution was decolourized with 10 g of activated carbon for 30 min. The solid phase was removed by filtration and 76.3 ml of t-octylamine (1.5 mol/mol) were added to the solution, containing 130.8 g of pravastatin. The suspension was cooled down and kept at -3ºC for 6 hours, after which the crystals of t-octylamine salt of Pravastatin were filtered. The product was washed with 300 ml of acetone. The crystals were dissolved in 650 ml of deionized water, 10 % sodium hydroxide was used to adjust the pH to 11.3 and 500 ml of butyl acetate were added. After 10 min of stirring, the layers were separated. Ion-exchange resin DIAION SK116 (H⁺) was added to the aqueous phase to reduce the pH to 8.2. The resin was removed by filtration and the water concentrate of Pravastatin sodium salt was decolourized with 8 g of activated carbon. The carbon was filtered and washed with 50 ml of deionized water. The aqueous solution was concentrated under vacuum to brix content of 31 %. Ethyl alcohol (39 ml) were dosed into the solution, and the mixture was added to 9.3 l of acetone. The mixture was stirred for 3 hours at temperature 21±2ºC and left to stay for 16 hours at 2ºC. The product was filtered, washed with 300 ml of acetone and dried under vacuum. 123.93 g of Pravastatin sodium salt was obtained with purity of 98.3 % (HPLC) calculated as dry substance, without ethanol. Water content: 1.4 %; ethyl alcohol: 0.06 %; acetone: 0.0035 %; pravastatin content: 99.71 %; 6-epi-pravastatin content: 0.14 %; pravastatin lactone: 0.024 %. Yield: 74.9 %.

### Example 2

300 g pravastatin sodium salt, obtained as in EXAMPLE 1 was dissolved in 1000 ml of deionized water and 40 ml of ethanol were added. The solution was frozen to temperature of -42°C and was sublimated under vacuum of 160-180 mbar at 10±1°C. The secondary drying was performed at temperature of 35°C until water content was reduced to 2 %.

298 g of pravastatin sodium salt was obtained with a crystalline structure and purity of 98.3 % (HPLC). Water content 1.8 %; ethyl alcohol 0.03 %; Pravastatin content: 99.84 % (calculated on dry basis without ethanol). Yield 99.17 %.

### Example 3

1500 ml of 10 % solution of diisooctylphosphate in butyl acetate were added to the aqueous solution, obtained as in Example 1, containing 110.3 g of pravastatin with pH 11.4. The mixture was stirred for 30 min at temperature of 22ºC. The butyl acetate layer was removed and to the aqueous solution of Pravastatin sodium salt were added 5.5 g of activated carbon and 38 g of aluminium oxide. The mixtrure was stirred for 40 min and the solid phase was removed by filtration. The cake was washed with 150 ml of deoinized water. The aqueous solution was concentrated under vacuum to brix content of 26 %. Ethyl alcohol (28 ml) were dosed to the resultant solution. The solution was frozen down to temperature of -43ºC and sublimated under vacuum of 170-185 mbar at 10±1ºC. The secondary drying was performed at temperature of 35ºC to water content less than 2 %.

111.7 g of pravastatin sodium salt with crystalline structure and purity of 97.7 % (HPLC) were obtained. Water content: 1.5 %; ethyl alcohol: 0.04 %; Pravastatin content: 99.23 % (calculated on dry basis without ethanol). Yield: 75.33 %.

## Claims

1. Method for the isolation and purification of pravastatin sodium, comprising extraction with organic solvent, reextraction with water, second extraction with organic solvent, precipitation of pravastatin, transformation of pravastatin into sodium salt and recovery of the salt, and particularly a method in which the fermentation broth is alkalized to pH 10.2, the mycelium is centrifuged, the resultant aqueous filtrate is acidified to pH 3-4, extracted with butyl acetate with continuous stirring, reextracted with water, and the resultant reextract is heated to temperature of 30-32°C, sodium hydroxide is added to reach a 0.5-4 % concentration of the hydroxide in the reextract; it is left to stay at these conditions for 85-90 min and is re-extracted again with butyl acetate in the presence of sodium chloride whose concentration in the reaction mixture is 10-20 %, the obtained butyl acetate extract is dried with disodium sulphate, decolourized and filtered and the precipitation of pravastatin is achieved by slowly adding to the filtrate 1.3-1.8 mol t-octylamine per mol pravastatin; the temperature of the reaction mixture is reduced to 0-5°C, the obtained crystal product is filtered, washed with acetone, dissolved in water and the pH of the mixture is raised to 10.5-13.0 to obtain pravastatin sodium salt, which is purified and recovered.

2. A method as in Claim 1, where the purification and recovery of pravastatin sodium salt is achieved by adding butyl acetate to its aqueous solution, the reagent mixture is stirred for 10-20 min, the layers are separated, the aqueous layer is separated and fused cathion exchange resin is added to it to pH 8.1-8.5, the resin is removed by filtration, the obtained filtrate is decolourized, concentrated under vacuum and a lower aliphatic alcohol 2 mol/mol pravastatin is added to the concentrate, it is transferred into acetone, so that the concentration of water in the resultant mixture is 2.0-3.0 %, it is stirred for 3-4 hours at temperature of 19-23°C, left for 16-20 hours to stay at temperature 5 ÷ -5°C, and the resultant crystals of pravastatin sodium salt are filtered, washed in acetone and dried.

3. A method as in Claims 1 and 2, where the recovery of Ha pravastatin sodium salt is achieved by dissolving the dried crystals of pravastatin sodium salt in water to obtain a 20÷35%solution, 1 mol of lower aliphatic alcohol alcohol per mol of pravastatin is added to the solution, after which the solution is lyophilized at freezing temperature of -42 ÷ -45°C, and temperature of sublimation 5-15°C nunder vacuum 100-250 mbar, and secondary drying is performed at temperature 35°C.

4. A method as in claims 1, 2 and 3, where as lower aliphatic alcohol for the recovery of pravastatin sodium salt methanol, ethanol, propanol, i-propanol, butanol, i-butanol, amyl alcohol, i-amyl alcohol can be used.

5. A method as in Claim 1, where for the purification and recovery of pravastatin sodium salt, its aqueous solution is extracted at temperature 10-30°C for 30-40 min with 10-40 % of liquid ionite to the quantity of pravastatin, dissolved in butyl acetate, then the butyl acetate layer is removed and the recovered aqueous layer is treated with aluminium oxide, decolourized with activated carbon, filtered, the filtrate is concentrated under vacuum and 2 mol of lower aliphatic alcohol per mol of pravastatin are added to it, the resultant solution is lyophilized at freezing temperature of -42 ÷ -45°C, temperature of sublimation 5-15°C under vacuum 100-250 mbar and temperature of secondary drying 35°C.

6. A method as in claims 1 and 5, where as liquid ionite for the extraction of pravastatin sodium salt can be used the following: Amberlit LA-1, Amberlit LA-2, Amberlit LA-3 and diisooctylphosphate.
